# EUROPEAN PATENT APPLICATION

(11) **EP 1 081 258 A1**
(43) Date of publication of application: **07.03.2001**
(21) Application number: 00115425.1
(22) Date of filing: 17.07.2000
(51) Int. Cl.: D01H 13/26, B65H 69/00, G01N 33/36

(54) **Yarn sampling apparatus**

(30) Priority: 20.08.1999 JP 23462199; 20.08.1999 JP 23462299
(71) Applicant: Murata Kikai Kabushiki Kaisha, Minami-ku, Kyoto-shi, Kyoto 601 (JP)
(72) Inventor: Hatakeyama, Yasunori, Kurita-gun, Shiga (JP); Honda, Noriaki, 1496-6 Kutsatsu-cho, Kutsatsu-shi, Shiga (JP); Miyano, Ken, Kameoka-shi, Kyoto (JP)
(74) Representative: Liedl, Christine, Dipl.-Chem.

(57) **Abstract**

Until now, when yarn was sampled, yarn ends had to be drawn manually from peg-held packages on a creel stand or the like and spliced together one at a time. Alternatively, the packages held on the creel stand had to be taken off the stand and placed on trays, either one by one or in multiple lots, and transported, with sampling performed as the packages are transported. The manual labor involved in drawing such yarns was troublesome, and manual labor was required to splice together the yarns. Additionally, it was difficult to draw the yarn ends from the packages.

In the present invention, a yarn sampling apparatus 1 draws and samples yarns Y directly from packages P held on pegs 5 of peg-equipped creel stands 3a, 3b. The pegs 5 are equipped with yarn end clamps 21, comprised as yarn end clamp members that lock the yarn ends.

## Description

### Field of the Invnetion

The present invention relates to a yarn sampling apparatus that samples yarns drawn from packages, and more specifically, to a yarn sampling apparatus adapted to sample yarn directly from peg-held packages without requiring that the package be moved onto a tray or the like.

### Background of the Invention

Conventionally, yarns from packages produced by a texturing machine such as a false twister are sampled before the packages are sent to the next stage of processing. During sampling, a short sample of the yarn is drawn out from each of the packages and knit on a knitting machine. The knit samples are then died and inspected. Yarn defects are easily detected by spotting dying deformities and variations in the degree to which dye takes to the yarn.

Recent years have seen an increasing demand for high quality yarn processing, and it has now become necessary to inspect all packages before sending them to the next step in the production process. Prior art apparatus have sampled packages by drawing a predetermined amount of yarn from one package and then splicing the yarn together with the yarn from another package.

Until now, sampling has entailed manually placing each package on a stand, manually drawing a length of yarn from the package, and then splicing the end of that yarn together with the yarn end of a yarn drawn from another package. Alternatively, yarn packages held on a stand or the like have been temporarily placed on trays in single package or several packages, and transported, with sampling taking place during transport.

In such cases, whether yarn ends are drawn directly from stand-held packages or tray-held packages in transit, since the yarn end is not always positioned at a predetermined location, grasping and drawing a predetermined length of yarn from the package can be a very complicated and troublesome operation. "Draw misses" where the yarn end is not successfully grasped, are not uncommon.

### Summary of the Invention

The object of the present invention is to provide a yarn sampling apparatus capable of arranging the yarn ends of a plurality of stand-held packages at fixed locations, thus enabling the yarn ends to be reliably grasped without human intervention, and capable of automatically splicing the grasped yarns drawn from the packages. Moreover, the object of the present invention is to provide a yarn sampling apparatus capable of grasping yarn ends directly from stand-held packages, such as packages on pegs, and sampling them automatically.

The present invention solves the problems set out above as follows. First, a yarn sampling apparatus that samples yarn drawn from a package samples yarn drawn directly from a peg-held package on a peg-equipped creel stand.

Further, the sampling apparatus is provided with a yarn end clamp that locks into position the yarn end of a package held on the pegs of the peg stand.

Further still, the yarn end clamp clamps the yarn end in two places. Still further, the yarn sampling apparatus samples yarns drawn directly from packages held on a plurality of pegs arranged in rows on a peg-equipped creel stand, and vertically positions a yarn joining device at a position corresponding to a peg that holds a package to be sampled when yarn is to be sampled from that package.

Also, the yarn joining device is held such that it can be slid freely along a vertically arranged rail, and the yarn joining device is elevated along the rail by a motor.

The yarn joining device is provided with a supply device that supplies colored yarn used as a marker between sampled yarns, and a standard yarn used as a start lot / end lot marker towards the yarn joining device, and the supply device holds the colored and standard yarns in a clamped state when the colored and standard yarns are not supplied, and releases the clamp to either the colored or standard yarns when the respective colored or standard yarn is supplied. This enables either colored or standard yarn to be reliably supplied to the yarn joining device.

### Brief Description of the Drawing

Figure 1 is a side-view of the yarn sampling apparatus of the present invention and peg-equipped package bearing creel stands.
Figure 2 is a side-view of the yarn sampling apparatus.
Figure 3 is a frontal-view of the yarn sampling appratus.
Figure 4 is a side-view of the yarn end clamp arranged on the end of the pegs.
Figure 5 is a planar view of the yarn end clamp.
Figure 6 is frontal view of the yarn end clamp.
Figure 7 is a side-view of the splicer.
Figure 8 is a planar view of the splicer.
Figure 9 is a frontal view of the splicer.

### Detailed Description of the Preferred Embodiments

Embodiments of the present invention will now be explained with reference to the accompanying drawings.

The structure of the yarn sampling apparatus of the present invention will now be explained.

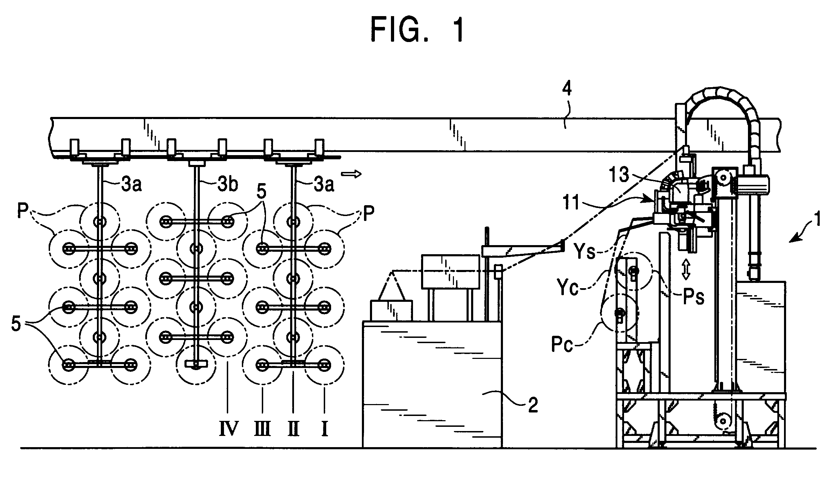

In Figure 1, a knitting machine 2 is arranged near a yarn sampling apparatus 1. A plurality of creel stands 3a, 3b are moveably attached to rails 4 that run along the ceiling.

Several rows and columns of pegs 5, 5 are arranged in the creel stands 3a, 3b, etc. The pegs 5 are staggered in the creel stands 3a, 3b. In the present embodiment, there are three rows and three columns of pegs 5 in each of the stands 3a, 3b.

Packages P are arranged and held on each of the pegs 5 in the creel stands 3a, 3b.

The creel stands 3a, 3b, each holding a plurality of packages P, are arranged so as to be moveable in the direction of the yarn sampling apparatus 1. When creel stand 3a or 3b is brought to a predetermined position in proximity to the yarn sampling apparatus 1, a splicer 11 of a yarn joining device 13 draws a yarn Y off a package P, splices it to a preceding yarn, and then leads it to knitting machine 2, where the yarn Y is knit.

The splicer 11 can move vertically, and is controlled so as to elevate or descend to a position corresponding to the height of the package P rows on the creel stands 3a, 3b.

When the packages P in the first column I of creel stand 3a are brought to the predetermined position in proximity to the yarn sampling apparatus 1, a predetermined length of yarn Y is drawn, one at time, from the package P in each row of the column I, and sampled.

After the yarns Y from all of the packages P in the column I have been sampled, the creel stand 3a is moved such that column II advances to the predetermined sampling position. The process is then repeated for column II, with the yarns Y from each row of packages in the column II drawn and sampled one at a time, before the next column III is advanced to the sampling position for sampling.

The sampled yarns Y are spliced by the splicer 11 of the yarn joining device 13 such that a single continuous yarn is formed. The continuous yarn is fed to and knit by the knitting machine 2, after which the knit yarn is died and inspected.

A colored yarn package Pc and a standard yarn package Ps are also arranged in the yarn sampling apparatus 1. After the yarn Y has been sampled from a predetermined number of packages P, a predetermined length of a colored yarn Yc is drawn from the colored yarn package Pc, and spliced to the sampled yarn Y. After the predetermined length of the colored yarn Yc is drawn, the yarn Y is once again drawn from the package P to be sampled, and that yarn Y is spliced to the tail end of the colored yarn Yc. The net effect is to insert the colored yarn Yc between individual sampled yarns Y, the colored yarn Yc to mark the boundaries therebetween.

Additionally, a standard yarn Ys from the standard yarn package Ps is drawn and spliced to the sampling yarn Y after the yarn Y for sampling have been drawn from the packages P. The standard yarn Ys is then knit to mark the end of a sampled group.

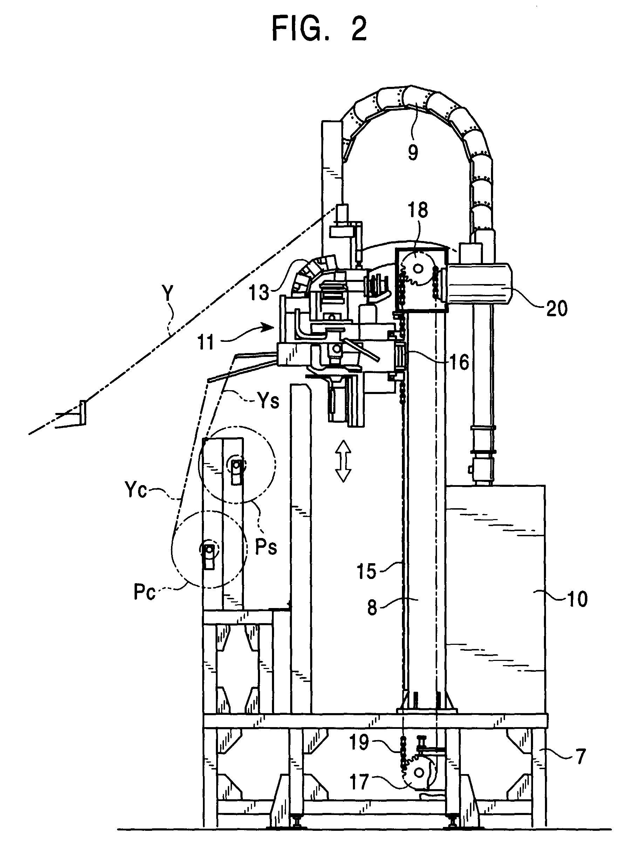

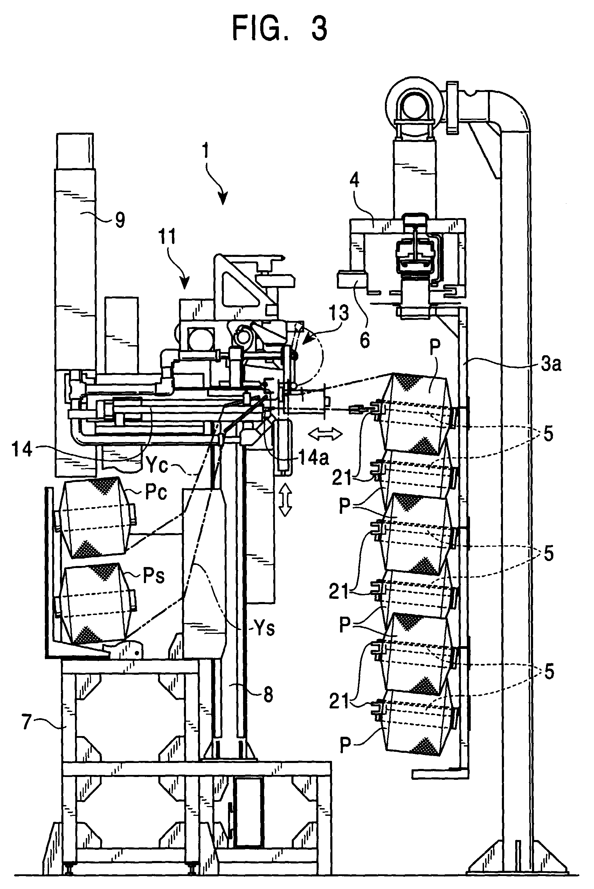

As shown in Figure 2 and Figure 3, a central support 8 stands on the base frame 7 of the yarn sampling apparatus 1, and an elevating member 16 of the splicer 11 is slidably held on a guide rail 15 of the support 8.

A lower sprocket 17 is arranged underneath the support 8 on the base frame 7, and an upper sprocket 18 is arranged at the top of the support 8. A chain 19 engaged its both ends with the elevating unit 16 is laid around the upper sprocket 18 and the lower sprocket 17.

The upper sprocket 18 is rotationally driven by an elevating motor 20, causing the splicer 11 to slide vertically along the guide rail 15.

In this manner, by elevating the splicer 11, which samples and splices the yarns Y drawn from the packages P, by elevating the chain 19 along the guide rail 15 with the elevating motor 20, the splicer 11 can be reliably elevating to a position corresponding to the peg 5 of the package P being sampled, enabling reliable sampling of the yarn Y.

Yarn end clampers 21 are provided at the ends of the pegs 5 of the creel stands 3a, 3b. The yarn end clampers 21 allow the ends of the yarns Y of the various packages P supported on the pegs 5 to be clamped and positioned.

A clamp head 14a is also provided on the splicer 11. The clamp head 14a can be moved back and forth in the direction of the packages P through the extension and contraction of a sample yarn clamp cylinder 14.

When the yarn Y is drawn from the package P by the splicer 11, the sample yarn clamp cylinder 14 is extended, moving the clamp head 14a towards the packages P. The yarn Y on the package P held in place at the tip of the peg 5 by a yarn end clamper 21 is then clamped by the clamp head 14a.

Next, the sample yarn clamp cylinder 14 is retracted, bringing the clamp head 14a with the clamped yarn Y back towards the splicer 11. This draws the yarn Y towards the splicer 11.

When a yarn clamping operation is performed, the splicer 11 is elevated to the height of the peg holding the package P from which the yarn Y is to be unwound, as explained above. After the creel stand 3a is brought to a horizontal position where the peg holding the package P to be unwound aligns with the position of the clamp head 14a of the splicer 11, locked in place by a position locking device 6, and brought to a halt.

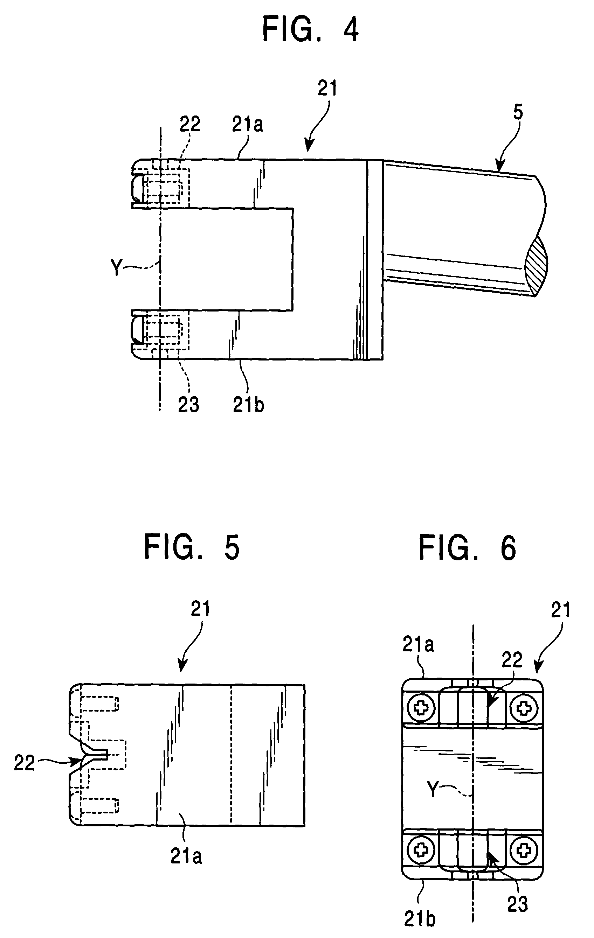

As can be seen in Figures 4 through 6, the yarn end clamper 21 that positions and locks the end of the yarn Y is arranged on the end of the pegs 5 is formed in a bracket shape when seen from the side-perspective view. The yarn end clamper 21 is principally comprised of an upper projecting wall 21a and a lower projecting wall 21b.

A cavity section is formed in the space between the upper projecting wall 21a and the lower projecting wall 21b, and an upper clamp part 22 and a lower clamp part 23 are formed on the almost center part of the upper projecting wall 21a and the lower projecting wall 21b, respectively. These upper and lower clamp parts 22, 23 function to grasp and clamp the yarn Y at two distinct points. By clamping the yarn Y at two points with the upper and lower clamp parts 22, 23, the clamp head 14a of the splicer 11 can then reliably clamp onto the section of the yarn Y spread between the upper and lower clamp parts 22, 23 and draw it away.

In other words, arranging the yarn end clamper 21 at the end of the pegs 5, in the form the yarn clamps, that clamps the end of the yarn Y drawn from the package P, the end of the yarn Y can be locked into position at a predetermined location. This then enables the yarn Y to be reliably and automatically drawn out by the slider using, for example, clamp head 14a.

Furthermore, since the yarn end clamper 21 is comprised as a mechanism that clamps the yarn Y in two places, the yarn Y can be reliably clamped. Still further, since the section of the Yarn Y spread between the upper and lower clamp parts 22, 23 can be grasped when the yarn Y is clamped by the clamp head 14a and the splicer 11 is withdrawn, the yarn Y can be easily clamped and withdrawn.

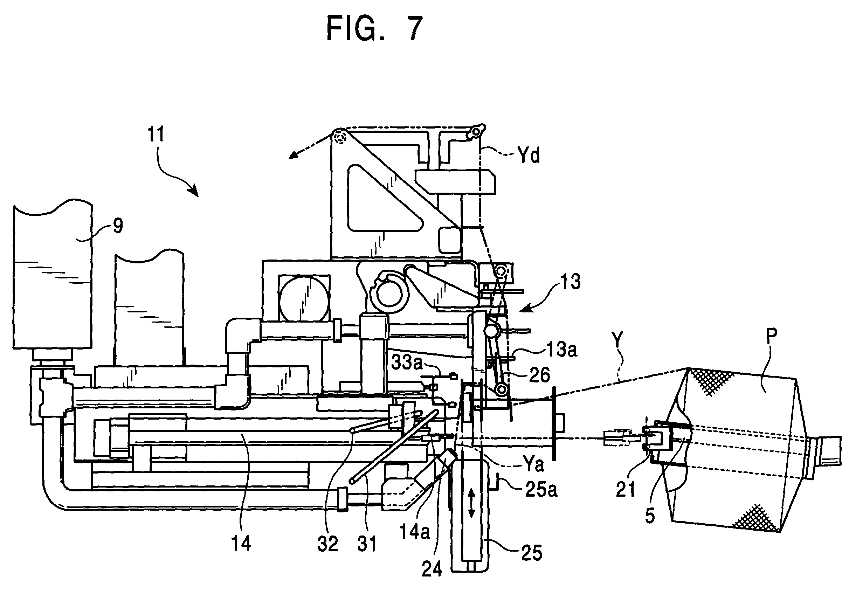

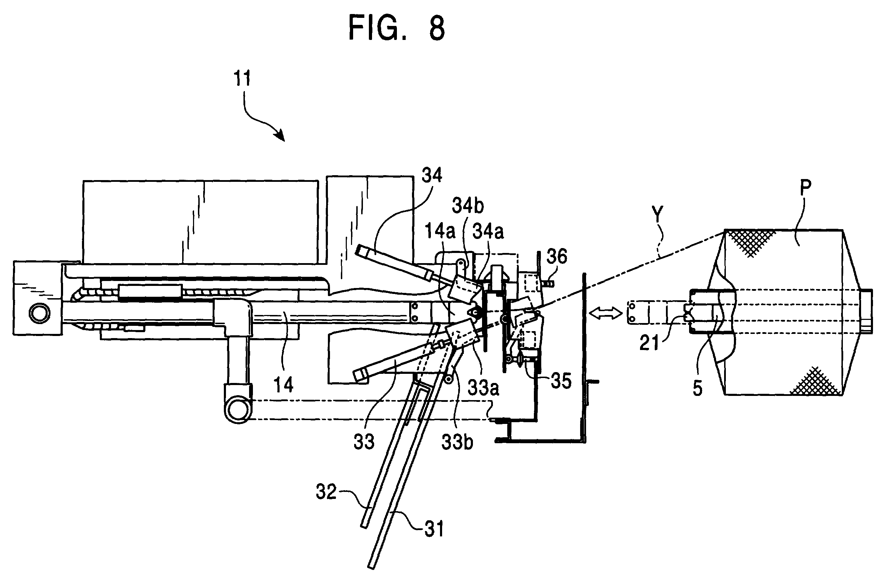

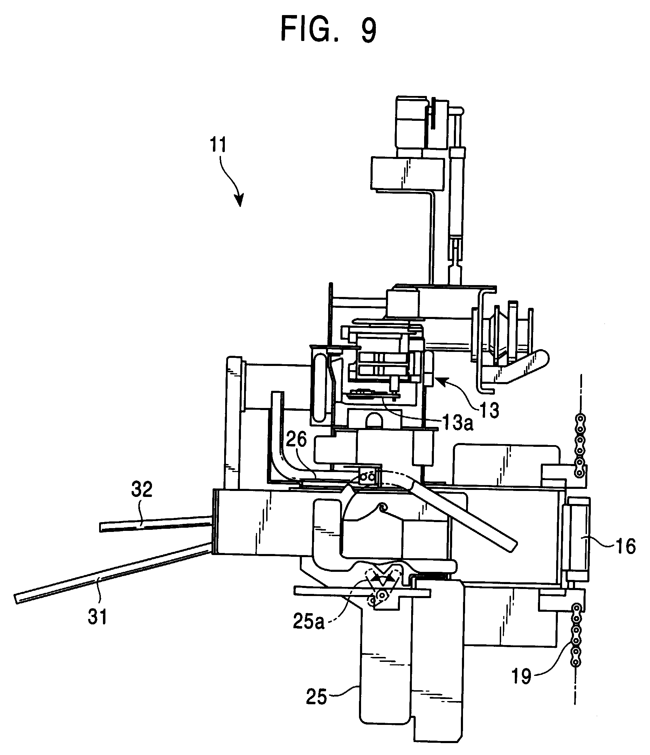

The splicer 11 will now be described with reference to Figure 7, Figure 8, and Figure 9.

As mentioned above, the splicer 11 is provided with the sample yarn clamp cylinder 14 that moves the clamp head 14a in the direction of the package P. Extending and retracting the sample yarn clamp cylinder 14 moves the clamp head 14a towards the package P, and retracting the sample yarn clamp cylinder 14 draws the yarn Y from the peg-held package P to the splicer 11.

The yarn Y drawn to the splicer 11 forms yarn path Ya, as shown in the diagram. The yarn Y is sucked into the outer yarn intake unit 24, removing the outer layer of yarn from the package P. The yarn Y removed from the package P is exhausted via an exhaust duct 9 into a yarn waste box 10 as shown in Figure 2. Removing the outer layer of yarn from the package P removes any yarn that may have been besmirched. Further, since the quality and characteristics of the yarn may change towards the end of the yarn take-up operation, and since occasionally yarn may be formed without a false twist after a yarn breakage has occurred, removing the outer layer of yarn removes any possible yarn irregularities that may have developed, and remove any sections of yarn which may potentially lack a false twist.

After the outer layer of yarn has been taken off the package P by the outer yarn intake unit 24, a yarn lifting device 25 is elevated, lifting the yarn Y. The lifted yarn Y is sucked in by vertically moveable suction pipe 26, the suction pipe 26 is pivotably raised, and then yarn joining device 13 joins the yarn Y with another yarn Yd from a different package P that has already been sampled.

Next, the yarn Y is cut by a cutter 25a between the suction pipe 26 and the outer yarn intake unit 24, separating the section of the yarn Y to be joined from the section that has been sucked in by the outer yarn intake unit 24.

A predetermined length of the yarn joined by the yarn joining device 13 is fed towards the knitting machine 2 and sampled. After the yarn Y has been drawn for sampling, it is cut and clamped by a clamp cutter 13a arranged at the end of the yarn joining device 13. The splicer 11 is then lowered, and sampling of a yarn Y from the next package P is initiated.

A colored yarn inserting cylinder 33 is arranged in the splicer 11 for supplying to and joining the colored yarn Yc, which is used as a marker to indicate the separation between different sampled yarns, at yarn joining device 13. A standard yarn inserting cylinder 34 is also arranged in the splicer 11 for supplying the standard yarn Ys, which is used to indicate the end of a knitting lot of sampled yarns.

A colored yarn clamp head 33a is arranged at the end of the colored yarn inserting cylinder 33. The colored yarn inserting cylinder 33 can be extended, thereby moving the colored yarn clamp head 33a that clamps the colored yarn Yc, which has been led to the splicer 11 via a colored yarn guide pipe 31, in the direction of the suction pipe 26.

A standard yarn clamp head 34a is arranged at the end of the standard yarn inserting cylinder 34. The standard yarn inserting cylinder 34 can also be extended, thereby moving the standard yarn clamp head 34a that clamps the standard yarn Ys, which has been led to the splicer 11 via the standard yarn guide pipe 32, in the direction of the suction pipe 26.

The colored yarn Yc is clamped by a clamp arm 33b on the colored yarn clamp head 33a. When the colored yarn Yc is supplied to the yarn joining device 13 and joined, the colored yarn inserting cylinder 33 is extended, and the colored yarn clamp head 33a is moved to the position of the lowered suction pipe 26.

When the colored yarn clamp head 33a reaches the position of the suction pipe 26, the colored yarn Yc, which is clamped by the colored yarn clamp head 33a, is released by a colored yarn clamp releasing cylinder 35, and the colored yarn Yc is sucked and held by the suction pipe 26.

Next, the suction pipe 26 is raised, and the colored yarn Yc is joined by the yarn joining device 13 in the same way as described with the sampled yarns Y.

Similarly, the standard yarn Ys, which is clamped by a clamp arm 34b on the standard yarn clamp head 34a, is supplied to the yarn joining device 13, and joined to the preceding sampled yarn by extending the standard yarn inserting cylinder 34, moving the standard yarn clamp head 34a to the position of the lowered downwardly pivoted suction pipe 26.

When the standard yarn clamp head 34a reaches the position of the suction pipe 26, the standard yarn Ys, which is clamped by the standard yarn clamp head 34a, is released by a standard yarn clamp releasing cylinder 36, and the standard yarn Ys is sucked and held by the suction pipe 26.

Next, the suction pipe 26 is raised, and the standard yarn Ys is joined by the yarn joining device 13 in the same way as described with the sampled yarns Y.

Thus, the colored yarn Yc and the standard yarn Ys are supplied between the sampled yarns Y, and the timing and amount of the colored yarn Yc and the standard yarn Ys supplied to the yarn sampling apparatus can be appropriately set.

The colored yarn Yc and the standard yarn Ys are supplied to the yarn joining device 13 by the colored yarn inserting cylinder 33 and the standard yarn inserting cylinder 34. When the colored yarn Yc or the standard yarn Ys are not supplied to the yarn joining device 13, the ends of the respective yarns Yc, Ys are clamped in place, and when the respective yarns Yc, Ys are supplied to the yarn joining device 13, the clamps of the ends of the respective yarns Yc, Ys are released. This allows a smooth, reliable supply of the colored yarn Yc and the standard yarns Ys to the yarn joining device 13.

In the yarn sampling apparatus 1 comprised as explained above, when the yarn Y is automatically drawn and sampled directly from the package P held on the peg 5 of the creel stands 3a, 3b having a plurality of peg rows, without having to first put the package P on a tray or the like, the yarn joining device 13 is elevated to the height of the peg 5 holding the package P being sampled. This allows the yarn end of the package P held in the creel stands 3a, 3b to be reliably grasped without human intervention, enabling sampling to be performed automatically.

This helps reduce the chances of triggering a sampling fault and reduces manual labor, simplifying the sampling process, and enabling that process to be fully automated.

It should be noted that the creel stands 3a, 3b need not hang from the ceiling, as disclosed in the embodiment described herein, but may be comprised as hand-pushed carts.

Comprised as described above, the present invention achieves the following.

First, since a yarn sampling apparatus samples yarn directly from a peg-held package on a creel stand, yarn can be drawn and sampled directly from the peg-held package without first having to transfer the package to a tray or the like. This obviates the necessity of manually loading the package onto a tray, and allows yarn to be sampled by reliably and automatically grasping the end of the yarn on the peg-held package without human intervention. Thus, sampling errors can be avoided, manual labor reduced, and the sampling process simplified, thereby allowing the sampling process to be completely automated.

Further, a yarn end clamp member that locks the yarn end of the peg-held package into position is provided, thereby enabling the yarn end to be firmly held at a fixed location. This then allows a clamp head or the like that clamps the yarn end to be employed for reliably and automatically drawing the yarn towards the yarn sampling apparatus.

Additionally, the yarn end clamp member clamps the yarn end in two places, thus enabling the yarn end to be reliably clamped. Since the yarn can be clamped between the two clamp points when it is withdrawn towards the yarn sampling apparatus for sampling, the process of clamping and withdrawing the yarn can be performed more easily.

Still further, since in a yarn sampling apparatus a yarn joining device is elevated to the height of a peg-held package held on a creel stand having multiple rows of pegs to be sampled when sampling is to be performed on a yarn drawn directly from the package, the yarn end on a package in a creel stand can be reliably grasped and automatically sampled without human intervention. Thus, sampling errors can be avoided, manual labor reduced, and the sampling process simplified, thereby allowing the sampling process to be completely automated.

Further still, the yarn joining device is held so that it can slide freely along a vertically arranged rail and is driven by a motor so as to elevate and descend along the rail, the splicer can be reliably elevated to the precise height of the peg of the package to be sampled, thus enabling sampling to be reliably performed.

Still further, the yarn joining device is provided with a supply device that supplies colored yarn used as a marker and standard yarn used to mark the end of a knitting lot towards the yarn joining device, and the supply device holds the colored, and standard yarns in a clamped state when the colored and standard yarns are not supplied, and releases the clamp to either the colored or standard yarns when the respective colored or standard yarn is supplied. This enables either colored or standard yarn to be reliably supplied to the yarn joining device.

## Claims

1. A yarn sampling apparatus that draws and samples yarns from a package characterized in that yarns are sampled directly from peg-held packages held in a peg-equipped creel stand.

2. The yarn sampling apparatus of claim 1 characterized in that the pegs of the creel stand are provided with yarn end clamp members that hold in position yarn ends of packages held by the pegs.

3. The yarn sampling apparatus of claim 2 characterized in that the yarn end clamp member clamps the yarn ends in two places.

4. A yarn sampling apparatus that draws and samples yarns from a package characterized in that
a yarn is sampled directly from a peg-held package on a creel stand having a plurality of peg rows, and
when the yarn is to be sampled, a yarn joining device in the yarn sampling apparatus is elevated to a position corresponding with the position of a peg that holds a package to be sampled.

5. The yarn sampling apparatus of claim 4 characterized in that the yarn joining device is held so as to freely slide along a vertically arranged rail, and the yarn joining device is driven by a motor so as to elevate and descend along the rail.

6. The yarn sampling apparatus of claim 5 characterized in that the yarn joining device is provided with
a supply device for supplying to the yarn joining device
a colored yarn that serves as a marker yarn, and
a standard yarn that serves as a start of lot / end of lot marker,
wherein the supply device holds the colored yarn and the standard yarn in clamped states when the yarn ends of the colored yarn and standard yarn are not supplied to yarn joining device,
and wherein the supply device releases either the yarn end of the colored yarn or the standard yarn when that yarn is supplied to the yarn joining device.
